# EUROPÄISCHE PATENTANMELDUNG

(11) **EP 1 674 866 A1**
(43) Veröffentlichungstag der Anmeldung: **28.06.2006**
(21) Anmeldenummer: 05026796.2
(22) Anmeldetag: 08.12.2005
(51) Int. Cl.: G01N 33/49, G01N 27/403

(54) **Vorrichtung zur Thermostatisierung einer Messzelle in einem Analysator und Messzelle, welche in einem Analysator austauschbar einsetzbar ist**

(30) Priorität: 23.12.2004 AT 21582004
(71) Anmelder: F.HOFFMANN-LA ROCHE AG, 4070 Basel (CH); Roche Diagnostics GmbH, 68305 Mannheim (DE)
(72) Erfinder: Krysl, Franz-Josef, Dr., 8020 Graz (AT); Huber, Wolfgang, 8502 Lannach (AT); Schneider, Friedrich, 8274 Buch (AT)
(74) Vertreter: Babeluk, Michael

(57) **Zusammenfassung**

Die Erfindung betrifft eine Vorrichtung zur Thermostatisierung einer Messzelle (1) in einem Analysator, zumindest bestehend aus der Messzelle (1) mit einem Messkanal (7), wobei im Messkanal (7) zumindest ein Sensorelement (10) angeordnet ist, und dem Analysator, welcher eine thermostatisierbare Auflagefläche (3) aufweist. Die Messzelle (1) ist in den Analysator austauschbar einsetzbar und zumindest in einem Kontaktbereich mit der thermostatisierbaren Auflagefläche (3) in Kontakt bringbar, wobei die Messzelle (1) eine zumindest in diesem Kontaktbereich im Wesentlichen planare Messzellenwand (2, 5) aufweist. Für eine rasche, reproduzierbare Thermostatisierung der in der Messzelle vorliegenden Medien und Sensorelemente ist eine wärmeleitfähige, elastische oder plastische Schicht (11) vorgesehen, welche zumindest im Kontaktbereich an zumindest einer Messzellenwand (2, 5) oder der thermostatisierbaren Auflagefläche (3) des Analysators haftet und beim Austausch der Messzelle (1) von der gegenüberliegenden thermostatisierbaren Auflagefläche (3) oder Messzellenwand (2, 5) weitgehend rückstandsfrei entfernbar ist.

## Beschreibung

Die Erfindung betrifft eine Vorrichtung zur Thermostatisierung einer Messzelle in einem Analysator, zumindest bestehend aus der Messzelle mit einem Messkanal, wobei im Messkanal zumindest ein Sensorelement angeordnet ist, und dem Analysator, welcher eine thermostatisierbare Auflagefläche aufweist. Die Messzelle ist in den Analysator austauschbar einsetzbar und zumindest in einem Kontaktbereich mit der thermostatisierbaren Auflagefläche in Kontakt bringbar, wobei die Messzelle eine zumindest in diesem Kontaktbereich im Wesentlichen planare Messzellenwand aufweist. Weiters betrifft dir Erfindung eine Messzelle, welche in einen Analysator austauschbar einsetzbar ist.

Es ist bekannt, dass viele Einrichtungen und Vorrichtungen, die Sensoren aufweisen, temperaturabhängige Signaleigenschaften zeigen. Diese Temperaturabhängigkeit ist je nach Sensortyp durch die Beeinflussung chemischer Prozesse, deren Gleichgewichtslage und/oder deren Kinetik bedingt, oder wird insbesondere bei elektrochemischen Sensoren durch Änderung physikalischer Eigenschaften, wie z.B. der elektrischen Leitfähigkeit, hervorgerufen.

Derartige Sensoren werden häufig in medizinischen Analysensystemen zur Bestimmung von Gaspartialdrucken von Blut, des pH-Wertes oder der Ionen- und Metabolitkonzentrationen von Körperflüssigkeiten eingesetzt. Insbesondere werden solche Sensoren in Blutgasanalysatoren eingesetzt, welche in der Diagnostik eine große Rolle spielen.

Während nun die Temperaturkoeffizienten der Sensoren relativ leicht durch entsprechende Kalibrationsmessungen ermittelt werden könnten, besteht ein Problem darin, dass beispielsweise bei der Bestimmung von Blutgasen und pH die Messgrößen (pO₂, pCO₂, pH) temperaturabhängig sind, und die für die Umrechnung erforderlichen Temperaturkoeffizienten der Probe nicht hinreichend genau bekannt sind. Eine Umrechnung der für eine Blutprobe, beispielsweise bei Raumtemperatur, gewonnenen Messwerte auf die bei Körpertemperatur (37°C) vorliegenden Messwerte ist daher ungenau.

Um die oben genannten Temperaturabhängigkeiten zu vermeiden, ist es bekannt, Messzellen mit Sensoren unter kontrollierten Temperaturverhältnissen - in Thermostaten - einzusetzen. Wenn Messzellen nach einer gewissen Einsatzzeit ausgetauscht werden sollen, ist für eine leichte Trennbarkeit zwischen der Messzelle einerseits und dem Thermostaten, der ein fester Bestandteil des Analysators ist, andererseits zu sorgen.

Im Allgemeinen werden die Messzellen in einer thermostatisierten Kammer des Analysators, welche auf konstanter Temperatur gehalten wird und zumeist aus einer Metalllegierung oder einem keramischen Werkstoff gefertigt ist, betrieben.

Besonders bei gelösten Gasen spielt die Temperatur der Probe zum Zeitpunkt der Messung eine wesentliche Rolle. Die Löslichkeit von Gasen in z.B. wässrigen Medien nimmt mit steigender Temperatur ab, sodass das gelöste Gas die Tendenz zeigt, aus der Lösung zu entweichen. Hierdurch wird ein höherer Wert gemessen. Umgekehrt wird bei einer niedrigeren Messtemperatur ein tieferer Wert ermittelt.

In der Medizin spielt die Analyse der Blutgasparameter eine wichtige Rolle, insbesondere im Rahmen einer Notfalluntersuchung. Unter dem Sammelbegriff Blutgasparameter versteht man die Werte für den Partialdruck des Sauerstoffs, des Kohlendioxides (der in einer physiologischen Probe gelösten Gase) und den pH-Wert der physiologischen Probe bzw. einer wässrigen Kontrolllösung.

Um die Situation im Körper des Patienten bestmöglich zu erfassen, wird hierbei bei einer Probentemperatur von 37°C gemessen. Auch wenn zwischen der Probennahme und dem Messzeitpunkt nur wenig Zeit vergeht, ist die Blutprobe deutlich abgekühlt und muss im Analysator sehr rasch wieder auf Körpertemperatur gebracht werden.

Die in der Messzelle eingesetzten Sensoren werden konstant auf der Messtemperatur gehalten, in diesem Fall auf 37°C. Dies ist notwendig, da der massive Heizblock (großes Gewicht) im Wärmeverlauf sehr träge reagiert und die Messzelle durch ihren Aufbau aus polymeren Werkstoffen (siehe beispielsweise EP 1 087 224 A2), die bekanntermaßen schlechte bis sehr schlechte Wärmeleiter sind, ebenfalls sehr träge bei einem Temperaturwechsel reagieren. Die thermostatisierten Gehäuseteile bestehen beispielsweise aus Polykarbonat, wobei Wandstärken von bis zu 5 mm vorgesehen sind, die ebenfalls den Wärmeübergangswiderstand vergrößern.

Auch wenn die Messzelle an eine oder mehrere thermostatisierte Oberflächen des Analysators gedrückt wird, ist die Berührung der thermostatisierten Oberfläche nur an wenigen Punkten und auf unreproduzierbare Art und Weise gegeben. Aus der EP 1 367 392 A1 ist in diesem Zusammenhang ein Analysengerät mit einer thermostatisierbaren Messzelle bekannt, die nicht näher beschriebene elektrochemische Elektroden aufweist. Die Messzelle wird mit Peltier-Elementen thermostatisiert, wobei zwischen den Peltier-Elementen und der Messzellenwand ein flaches, thermisch leitendes Verteilerelement angeordnet ist. Die so gewählte Thermostatisierung kommt daher aufgrund der unvermeidbaren Luftspalte einem Luftbad gleich, sodass der Wärmeübergang in der Hauptsache durch die Dicke des schlecht wärmeleitenden polymeren Werkstoffes um den elektrochemischen Sensor und der verbleibenden Luftspalte zur thermostatisierten Oberfläche begrenzt wird.

Die Folge einer solchen Anordnung ist eine verzögerte Temperaturannahme des Sensors und der Probe. Damit ist die Messbereitschaft bei der jeweiligen Solltemperatur verlangsamt. Um in der Praxis diese Phase möglichst kurz zu halten, wird die Probe in einer Vorwärmstrecke, die der Messzelle vorgeschaltet ist, auf etwa die Solltemperatur gewärmt. Somit wird die Temperaturannahme der Sensoren bzw. der Proben am Messort wesentlich erleichtert und die erforderliche Solltemperatur rascher erreicht.

Manche Sensoren beinhalten Bestandteile, welche jedoch bei der erforderlichen Betriebstemperatur eine eingeschränkte Lebensdauer aufweisen, wie z.B. Enzyme, die die erforderlichen Sensorreaktionen am Messort ermöglichen. Wenn diese Enzyme durch längere Temperatureinwirkung teilweise oder gänzlich zerstört sind, das heißt, in ihrer Aktivität vermindert sind oder sogar inaktiviert wurden, kann der Sensor nicht mehr verwendet werden. Die Verwendungsdauer dieser enzymhaltigen Sensoren ist dadurch üblicherweise verkürzt.

In diesem Zusammenhang ist aus der US 5,046,496 A eine Sensoreinrichtung zur Messung der Blutgasparameter pH, pCO₂ und pO₂ bekannt geworden, bei welcher die einzelnen Elektroden mit Hilfe einer Dickfilmtechnik auf ein rechteckiges Trägerplättchen aus nicht leitender Keramik aufgetragen sind. Das Trägerplättchen mit den Messelektroden wird in das Gehäuse einer Durchflusszelle eingeklebt. Auf dem Trägerplättchen befinden sich weiters ein Temperatursensor und ein Heizelement, um die für die Messung notwendige Temperatur bereitzustellen und zu regeln. Nachteilig dabei ist der zusätzliche Aufwand, der mit der Integration eines Heizelementes sowie eines Temperaturmesselementes direkt in die Messzelle verbunden ist.

Aus der US 2003/0220583 A1 ist ein portables Diagnosesystem bekannt, bei welchem das Heizelement ebenfalls direkt in den Sensorchip mit den einzelnen Elektroden integriert ist, wobei der Sensorchip zur Temperaturmessung von einem IR-Sensor berührungslos abgetastet wird.

Wird bei austauschbaren Sensoren das Heizelement nicht im jeweiligen Sensorgehäuse integriert, sondern zentral im Analysator angebracht, so ist eine deutlich kostengünstigere und einfachere Fertigung der Sensoren/Messzellen möglich, da zu deren Fertigung weniger Bauteile bzw. Prozessschritte benötigt werden.

Schließlich ist aus der US 2003/0057108 A1 ein Verfahren zu schnellen Hydratisierung und Erwärmung von chemischen, elektrochemischen und biochemischen Sensoren bekannt. Die Sensorkassette besteht aus einem Unterteil aus Kunststoff, in welchem die Sensoren angeordnet sind und einer Abdeckplatte aus Metall, welche auch zur Übertragung von Wärme in die Sensorkassette verwendet werden kann. Zu diesem Zweck ist die Abdeckplatte mit entsprechenden Heiz-oder Kühlelementen, beispielsweise einem Peltier-Element, kontaktiert.

Aufgabe der Erfindung ist es, ausgehend vom dargelegten Stand der Technik eine Vorrichtung zur Thermostatisierung einer in einen Analysator einsetzbaren Messzelle vorzuschlagen, welche möglichst unter Verzicht auf eine Vorwärmstrecke für eine rasche, reproduzierbare Thermostatisierung der in der Messzelle vorliegenden Sensoren, der Kalibriermedien, der Kontrollmedien und der Probe geeignet ist, wobei ein problemloser Wechsel der Messzelle bei einer Fehlfunktion oder nach Ablauf der Einsatzzeit gewährleistet sein soll.

Diese Aufgabe wird erfindungsgemäß dadurch gelöst, dass zur Verbesserung des Wärmeüberganges auf die Messzelle eine wärmeleitfähige, elastische oder plastische Schicht vorgesehen ist, welche zumindest im Kontaktbereich an zumindest einer Messzellenwand oder der thermostatisierbaren Auflagefläche des Analysators haftet und beim Austausch der Messzelle von der gegenüberliegenden thermostatisierbaren Auflagefläche oder Messzellenwand weitgehend rückstandsfrei entfernbar ist und/oder dass die Messzellenwand, an deren dem Messkanal zugewandten Innenseite das zumindest eine Sensorelement angeordnet ist, zumindest im Kontaktbereich mit der thermostatisierbaren Auflagefläche des Analysators aus einem wärmeleitfähigen Metall oder einer Metalllegierung besteht.

Durch diese erfindungsgemäßen Maßnahmen (wärmeleitfähige Schicht bzw. metallische Messzellenwand), welche auch kombiniert werden können, wird der Wärmedurchgangswiderstand von der Wärmequelle, der thermostatisierten Auflagefläche des Analysators, bis zur Sensor- bzw. Probenebene wesentlich minimiert. Weiters kann eine schadhafte Messzelle oder eine Messzelle am Ende deren Standzeit problemlos ausgetauscht und einfach durch eine neue Messzelle ersetzt werden, ohne die thermischen Gegebenheiten zu ändern.

Gemäß einer Ausführungsvariante der Erfindung kann die Messzelle zwei oder mehrere planare Messzellenwände aufweisen, die jeweils unter Zwischenlage einer wärmeleitfähigen, elastischen oder plastischen Schicht, welche zumindest im Kontaktbereich an der jeweiligen Messzellenwand oder der thermostatisierbaren Auflagefläche des Analysators haftet und beim Austausch der Messzelle von der jeweiligen gegenüberliegenden thermostatisierbaren Auflagefläche oder Messzellenwand weitgehend rückstandsfrei entfernbar ist, an einer thermostatisierbaren Auflagefläche des Analysators anliegen.

Bei einer metallischen Messzellenwand, beispielsweise aus Kupfer oder Aluminium, kann aufgrund der hohen Festigkeit metallischer Werkstoffe die Messzellenwand sehr dünn ausgeführt sein, beispielsweise in Form eines Blättchens mit einer Dicke von vorzugsweise bis zu 2000 µm, besonders bevorzugt bis zu 1000 µm. Dadurch, dass die Metallschicht sehr dünn ausgebildet wird, kann auch die Wärmekapazität minimiert werden, so dass die gewünschte Messzellentemperatur schneller erreicht wird.

Bei Verwendung elektrochemischer Sensoren können die elektrisch leitenden Strukturen allerdings nicht direkt auf ein Metallblättchen aufgebracht werden. Zur Vermeidung von Kurzschlüssen ist daher bei einer Messzellenwand aus Metall oder einer Metalllegierung der zumindest eine elektrochemische Sensor unter Zwischenlage einer Zwischenschicht, welche eine elektrischen Isolierung bewirkt, an der dem Messkanal zugewandten Seite der planaren Messzellenwand angeordnet. Zur elektrischen Isolierung wird eine sehr dünne, vorzugsweise bis zu 100 µm dicke, besonders bevorzugt bis zu 10 µm dicke, elektrisch nicht-leitende Schicht, beispielsweise eine Kunststoffschicht, auf die Messzellenwand aus Metall oder einer Metalllegierung aufgebracht. Diese kann durch eine dünne Schicht, z.B. aus einem Polymer, gebildet werden, die durch Laminierung oder Beschichten aufgetragen wird. Beispiele zur Erzeugung elektrisch nicht-leitender Kunststoffschichten sind z.B. Kunststoff-Folien aus Polykarbonat, Polyester oder Polyvinylchlorid, die auf das Metallblättchen aufgeklebt werden oder Polykarbonat-und Polyesterlacke, die auf das Metallblättchen aufgebracht werden.

Auch bei Verwendung von Sensortypen, welche nicht auf elektrochemischen Technologien beruhen bzw. keine elektrischen Ableitungen benötigen, weist die erfindungsgemäße Ausführung der Messzellenwand, an deren dem Messkanal zugewandten Innenseite das zumindest eine Sensorelement angeordnet ist, Vorteile auf, wenn diese zumindest im Kontaktbereich mit der thermostatisierbaren Auflagefläche des Analysators aus einem wärmeleitfähigen Metall oder einer Metalllegierung bestehen. Solche Sensortypen sind beispielsweise Sensoren, welche auf optischen Technologien basieren oder auf der Bestimmung intrinsischer Eigenschaften der Probenflüssigkeit, beispielsweise deren elektrischer Leitfähigkeit, beruhen, da auch solche Sensoren unter möglichst definierten Temperaturbedingungen betrieben werden müssen, um zu möglichst exakten und reproduzierbaren Analytbestimmungen zu gelangen.

Da es für eine rasche und reproduzierbare Thermostatisierung wesentlich ist, neben den in die Messzelle eingebrachten Medien, wie Kalibriermedien, Kontrollmedien oder Probenflüssigkeiten, auch die in der Messzelle vorliegenden Sensoren schnellstmöglich auf die erforderliche Betriebstemperatur zu bringen, ist die erfindungsgemäße Ausführung vorteilhaft, da hier der Wärmetransfer zu den Sensoren besonders rasch und reproduzierbar erfolgen kann. Insbesondere ist diese erfindungsgemäße Ausführungsform besonders vorteilhaft gegenüber Ausführungsformen, bei welchen eine Metallschicht zum Wärmetransfer an der den Sensoren abgewandten Messzellenwand angebracht ist, da dort die Thermostatisierung der Sensoren durch die beschränkte Wärmeleitfähigkeit des sich zwischen Messzellenwand und Sensoren befindlichen Mediums langsamer erfolgt. Durch die Tatsache, dass unterschiedliche in der Messzelle vorliegende Medien, beispielsweise unterschiedliche Kalibriermedien, Kontrollmedien oder Probenflüssigkeiten, unterschiedliche Wärmeleitfähigkeiten aufweisen, erfolgt der Wärmetransfer bei solchen Anordnungen nicht exakt reproduzierbar. Dies ist insbesondere bei gasförmigen Kalibriermedien der Fall, wie sie beispielsweise bei Sensoren zur Bestimmung gasförmiger Analyten wie Sauerstoff eingesetzt werden können. Im Gegensatz dazu erfolgt entsprechend der erfindungsgemäßen Lösung der Wärmetransfer zwischen der thermostatisierbaren Auflagefläche des Analysators und den Sensoren in vorteilhafter Weise entlang definierter Schichten, deren Wärmeleitfähigkeiten bekannt sind.

Messzellenwände, an deren dem Messkanal zugewandten Innenseite das zumindest eine Sensorelement angeordnet ist und welche zumindest im Kontaktbereich mit der thermostatisierbaren Auflagefläche des Analysators aus einem wärmeleitfähigen Metall oder einer Metalllegierung bestehen, müssen im Sinne der vorliegenden Anmeldung nicht unbedingt als durchgehende Metallschichten ausgebildet sein. So sind im Rahmen der vorliegenden Erfindung auch Ausführungsformen umfasst, bei welchen die Metallschicht Aussparungen in bestimmten Teilbereichen, beispielsweise in Form von Löchern oder Gitterstrukturen, aufweist. Solche Ausführungsformen sind beispielsweise dann besonders vorteilhaft, wenn optische Sensortechnologien eingesetzt werden, da durch solche räumlich begrenzten Aussparungen in der Metallschicht ein Einstrahlen von Licht zu den Sensoren hin bzw. ein Auslesen des von den Sensoren emittierten Lichts ermöglicht wird, ohne den erfindungsgemäßen Wärmetransfer zu den Sensoren und zum Messkanal wesentlich zu verschlechtern, insbesondere wenn die Aussparungen möglichst geringe Flächen der Metallschicht einnehmen. Solche optischen Sensortechnologien sind beispielsweise in "Fluorescent optical sensors for critical care analysis" J.K. Tusa, M.J.P. Leiner; Ann Biol Clin 2003, 61:183-191 beschrieben. Auch bei elektrochemischen Sensortechnologien können solche Metallschichten mit Aussparungen in bestimmten Ausführungsformen vorteilhaft eingesetzt werden, da durch solche Aussparungen in der Metallschicht ein Durchkontaktieren der Sensoren auch durch die erfindungsgemäße Metallschicht möglich ist. Hierzu ist es aber weiterhin notwendig, eine entsprechende elektrische Isolierung der Bauteile zu gewährleisten, beispielsweise durch einen Luftspalt zwischen Metallschicht und elektrischer Ableitung des Sensors oder durch das Aufbringen einer isolierenden Schicht auf die Oberfläche der Metallschicht im Bereich der Aussparungen oder auf die elektrische Ableitung des Sensors.

Die Zwischenlage einer Zwischenschicht zwischen einer Messzellenwand aus Metall oder einer Metalllegierung und dem Messkanal bzw. den dem Messkanal zugewandeten Sensoren kann bei allen Sensortypen, nicht nur bei elektrochemischen Sensoren, vorteilhaft sein. Eine solche Zwischenschicht kann beispielsweise zu einer vorteilhaften Beeinflussung der Oberflächeneigenschaften des Messkanals, beispielsweise einer verbesserten Hydrophilie dessen Oberflächen, zur Erzielung verbesserter Oberflächeneigenschaften, insbesondere Haftungseigenschaften, für den Aufbau weiterer Schichten, zur Verbesserung des Korrosionsschutzes der darunter liegenden Metallschicht oder zur Vermeidung unerwünschter Reaktionen zwischen Metallschicht und der im Messkanal befindlichen Flüssigkeiten dienen.

In einer weiteren bevorzugten Ausführungsform ist die erfindungsgemäße Vorrichtung zur Thermostatisierung einer Messzelle in einem Analysator bzw. die Messzelle selbst derartig ausgebildet, dass das zumindest eine Sensorelement der Messzelle als optisches Sensorelement ausgebildet ist und unter Zwischenlage einer Zwischenschicht, welche optisch transparent ist, an der Messzellenwand, welche beispielsweise aus einem Metall oder einer Metalllegierung besteht, angeordnet ist. Beim Einsatz optischer Sensortechnologien kann eine Zwischenschicht zwischen Sensoren und der benachbarten, dem Wärmetransfer dienenden Metallschicht auch vorteilhafterweise derartig ausgebildet sein, dass sie optisch transparent ist. Ferner kann sie so ausgebildet sein, dass sie Lichtleiterfunktionen übernehmen kann. Eine solche Zwischenschicht kann insbesondere dazu eingesetzt werden, Anregungslicht zu den Sensoren bzw. von den Sensoren emittiertes Licht zu geeigneten Detektoren hinzuleiten. Solche Ausführungsformen sind insbesondere für optische Verfahren und Anordnungen, bei welchen das Anregungslicht bzw. das emittierte Licht seitlich ein- bzw. ausgestrahlt werden soll und wie sie beispielsweise in der EP 0 793 090 B1 beschrieben sind, vorteilhaft. Auch Kombinationen einer solchen lichtleitenden Zwischenschicht mit einer Metallschicht, welche im Bereich der Sensoren Aussparungen aufweist, sind in vorteilhafter Weise möglich, beispielsweise bei einer Detektion der von den Sensoren emittierten Strahlung senkrecht zur Einstrahlungsrichtung des Anregungslichtes.

Gemäß einer vorteilhaften Ausführungsvariante der Erfindung weist die wärmeleitfähige, elastische oder plastische Schicht zumindest an deren freien Oberfläche eine Struktur, beispielsweise in Form von Streifen, Noppen oder dgl., auf.

Zur Verbesserung der Wärmeleitung kann die wärmeleitfähige, elastische oder plastische Schicht Partikel aus einem gut wärmeleitenden Material, vorzugsweise keramische Partikel, aufweisen.

Weiters kann die Vorrichtung, bestehend aus Messzelle und Heiz- bzw. Kühlelement des Analysators bzw. dessen thermostatisierbarer Auflagefläche miniaturisiert werden, sodass bei einer geringeren Masse bzw. einer geringeren Baugröße die Solltemperatur rascher und ohne Verwendung einer Vorwärmstrecke erreicht werden kann.

Um den Wärmeübergang von der Wärmequelle zur Probe selbst bei Verwendung einer wärmeleitfähigen, elastischen oder plastischen Schicht weiter zu verbessern, kann die im Wesentlichen planare Messzellenwand aus einem gut wärmeleitenden Material, vorzugsweise aus Keramik, aus einem Metall oder einer Metalllegierung, ausgeführt sein. Als Materialien eignen sich z.B. Keramiken aus diversen Oxyden und Nitriden, wie z.B. Aluminiumoxyd, Aluminiumnitrid, Zirkoniumoxyd, Zirkoniumnitrid, Boroxyd oder Bornitrid, etc. oder Metalle wie Kupfer oder Aluminium, etc.

Gemäß einer vorteilhaften Ausführungsvariante kann die Messzelle zweiteilig ausgebildet sein und bei einseitiger Thermostatisierung aus einem durch die zumindest im Kontaktbereich zur thermostatisierbaren Auflagefläche planare Messzellenwand gebildeten Gehäuseunterteil aus einem gut wärmeleitenden Material und einem thermisch isolierenden Gehäuseoberteil bestehen, welches unter Zwischenlage von Dichtelementen den Messkanal begrenzt.

Die erfindungsgemäße Vorrichtung weist eine Reihe von Vorteilen auf:
- Der Wärmeübergang, der damit erzielbar ist, ermöglicht bei geeigneter Sensorkonstruktion - insbesondere in Zusammenhang mit geringen thermischen Massen - eine Geschwindigkeit des Temperaturanstiegs der im Messkanal vorliegenden Probe von etwa 5°C/s. Der Sensor erfährt durch seine Ankopplungstechnik eine wesentlich geringere Abkühlung durch eine kühle Probe und muss daher nicht die vollständige Aufwärmphase durchmachen.
- Wird die Wärmequelle für die thermostatisierbare Auflagefläche als Peltierelement ausgebildet, können die Messzelle und die darin angeordneten Sensoren auch gekühlt werden. Hierdurch können Sensorsysteme z.B. während einer Bereitschaftsphase kühler gehalten und bei Bedarf innerhalb weniger Sekunden auf Betriebstemperatur gebracht werden.
- Durch die kleineren zu thermostatisierenden Massen ergibt sich ein schnelleres Temperatureinstellverhalten von Sensor und Probe bei geringerem Energieverbrauch.
- Lästige Wartezeiten können so deutlich vermindert und Messwerte schneller erhalten werden, bei einer gleichzeitigen Lebensdauerverlängerung temperaturempfindlicher Sensoren.
- Bei geeigneter Konstruktion des Gesamtsystems sind die zu erwärmenden Massen gering, sodass der Energieaufwand deutlich geringer wird als bei konventionellen Systemen. Aufgrund der kleineren Geometrie können die zu isolierenden Flächen kleiner gehalten werden.
- Der durch die erfindungsgemäße Vorrichtung bedingte geringere Isolationsaufwand benötigt weniger Platz um die Messzelle. Zusammen mit einem geringeren Energieaufwand ist auch der Raumbedarf bei der Bereitstellung dieser Energie geringer (z.B. Auslegung des Netzteiles). Insgesamt wird die Verlustwärme geringer, sodass Bauabstände innerhalb des Gerätes kleiner gehalten werden können. Alle genannten Eigenschaften sind vorteilhafte Voraussetzungen für eine Miniaturisierung des Gesamtsystems.

Die Erfindung wird im Folgenden anhand von schematischen Darstellungen und Diagrammen näher erläutert. Es zeigen:
- Fig. 1: eine erfindungsgemäße Vorrichtung zur Thermostatisierung einer in einen Analysator einsetzbaren Messzelle in einer Schnittdarstellung normal zur Flussrichtung der Probe;
- Fig. 2 bis Fig. 4: unterschiedliche Ausführungsvarianten der erfindungsgemäßen Vorrichtung in einer Schnittdarstellung gemäß Fig. 1;
- Fig. 5: eine Explosionsdarstellung der Vorrichtung gemäß Fig. 4;
- Fig. 6: eine Schnittdarstellung der Vorrichtung gemäß Fig. 3 parallel zur Flussrichtung der Probe;
- Fig. 7: ein Detail der Messzelle in einer vergrößerten Darstellung; sowie
- Fig. 8: ein Messdiagramm erstellt mit der erfindungsgemäßen Vorrichtung.

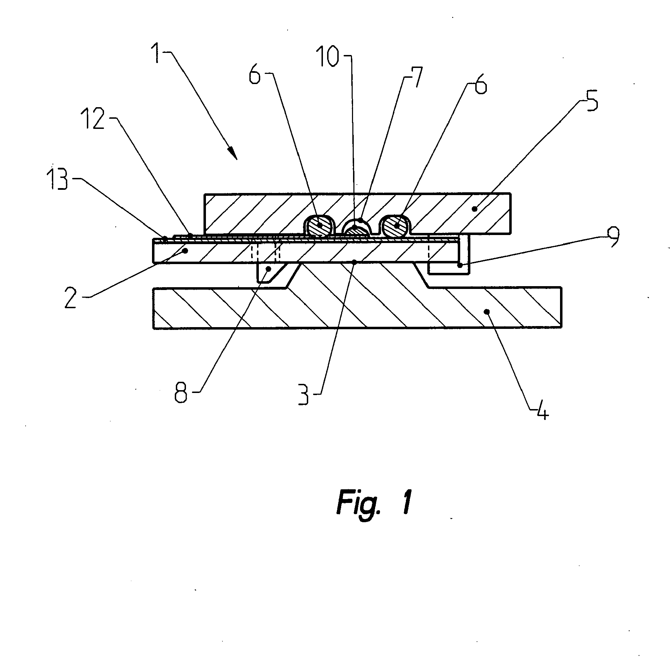

Die in Fig. 1 dargestellte Vorrichtung zur Thermostatisierung einer in einen (hier nicht weiter dargestellten) Analysator einsetzbaren Messzelle 1, weist zumindest eine im Wesentlichen planare Messzellenwand 2 auf, welche mit einer thermostatisierbaren Auflagefläche 3 des Analysators in Kontakt bringbar ist. Die Auflagefläche 3 dient zur gleichmäßigen Übertragung der von einem Heiz- bzw. Kühlelement 4 (z.B. Peltierelement) zur Verfügung gestellten Wärmeenergie. Die Messzelle 1 ist im dargestellten Beispiel als zweiteilige Durchflusszelle ausgeführt, welche die Probe in einer Flussrichtung normal auf die Zeichnungsebene durchströmt. Die planare Messzellenwand 2 ist als Gehäuseunterteil ausgeführt, besteht aus einem gut wärmeleitenden Material und begrenzt mit einem thermisch isolierenden Gehäuseoberteil 5 - unter Zwischenlage von Dichtelementen 6 - den Messkanal 7. Die beiden Gehäuseteile 2, 5 sind mit Hilfe der Rastelemente 8, 9 verbunden. Im Messkanal 7 ist zumindest ein Sensorelement 10, beispielsweise ein elektrochemischer Sensor, angeordnet. Im dargestellten Beispiel besteht die planare Messzellenwand 2 aus einem Metall oder einer Metalllegierung, sodass ein guter Wärmeübergang zu den Sensorelementen 10 und der Probe im Messkanal 7 gewährleistet ist. Bei Verwendung elektrochemischer Sensoren sind diese und deren Leiterbahnen 12 zur Ableitung der Sensorsignale unter Zwischenlage einer Zwischenschicht 13, welche eine elektrische Isolierung bewirkt, an der Messzellenwand 2 angeordnet.

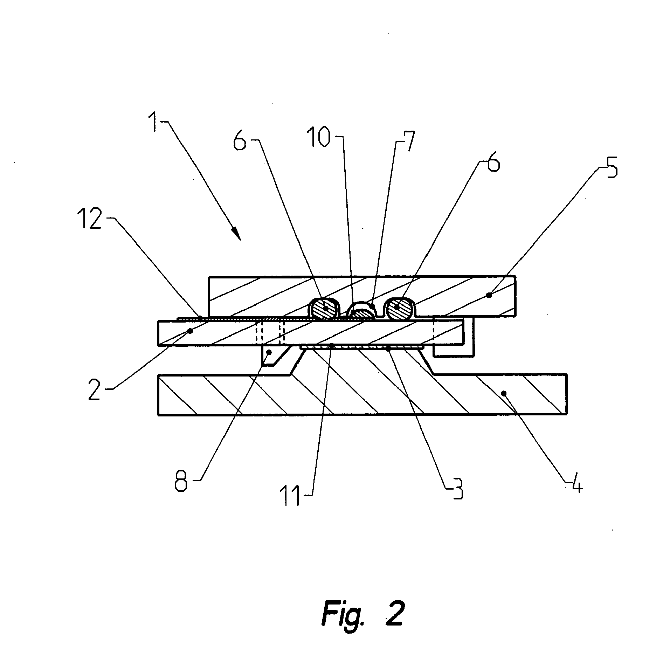

Bei der Ausführungsvariante gemäß Fig. 2 besteht die planare Messzellenwand 2 aus Kunststoff oder einem elektrisch nicht-leitenden anorganischen Material, wie z.B. Keramik, sodass hier keine Zwischenschicht 13, welche eine elektrische Isolierung bewirkt, notwendig ist. Zwischen der planaren Messzellenwand 2 und der thermostatisierbaren Auflagefläche 3 des Analysators ist eine wärmeleitfähige, elastische oder plastische Schicht 11 vorgesehen, welche an einer der beiden benachbarten Flächen 2 oder 3 haftet und beim Austausch der Messzelle von der anderen der beiden benachbarten Flächen 3 bzw. 2 rückstandsfrei entfernbar ist. Bevorzugt ist die Schicht 11 am planaren Gehäuseunterteil 2 fixiert, sodass diese bei jedem Wechsel der Messzelle 1 erneuert wird. Die wärmeleitfähige, elastische oder plastische Schicht 11 besteht bevorzugt aus einem wärmeleitfähigen Silikonmaterial und kann beispielsweise in-situ auf der planaren Messzellenwand 2 oder der thermostatisierbaren Auflagefläche 3 des Analysators ausgehärtet sein. Das Aufbringen der wärmeleitfähigen, elastischen oder plastischen Schicht 11 kann auch mittels Siebdruck, Schablonendruck oder ähnlicher Verfahren erfolgen.

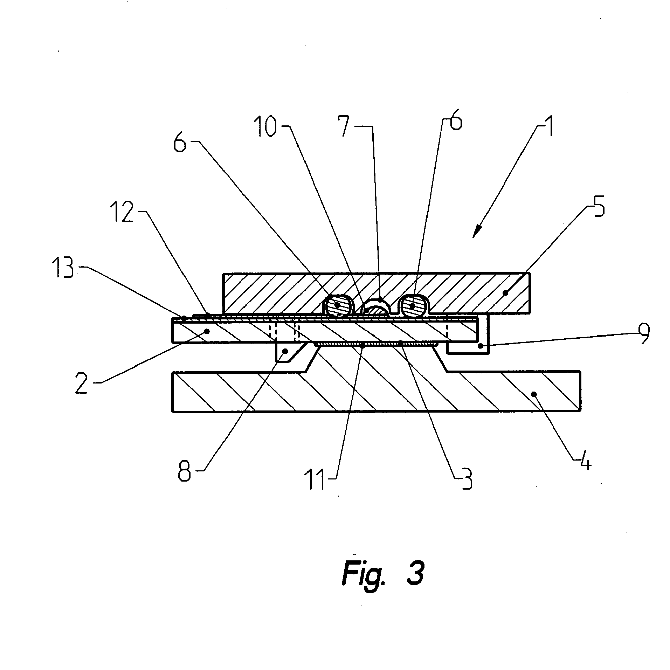

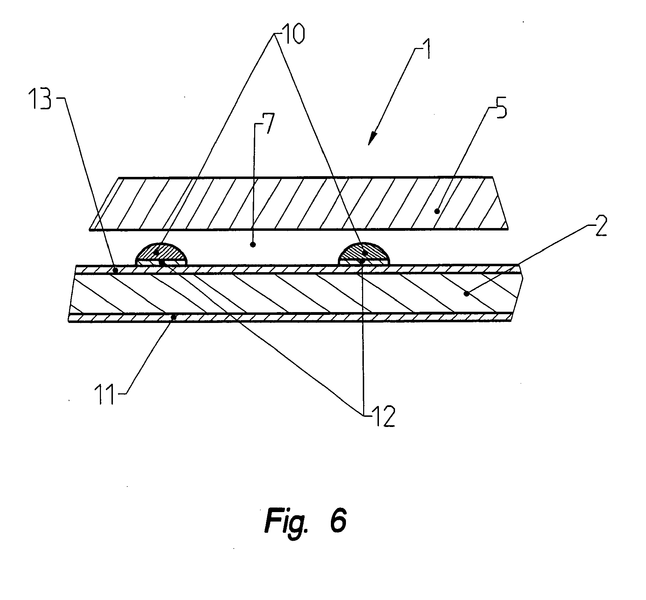

Die Ausführungsvariante gemäß Fig. 3 kombiniert die Vorteile der in den Fig. 1 und 2 dargestellten Varianten. Die planare Messzellenwand 2 besteht hier aus Metall oder einer Metalllegierung, wobei zur weitern Verbesserung des Wärmeübergangs eine wärmeleitfähige, elastische oder plastische Schicht 11 zwischen der Auflagefläche 3 des Analysators und der Messzellenwand 2 vorgesehen ist. In Fig. 6 ist ein Längsschnitt einer Messzelle 1 gemäß dieser Ausführungsvariante im Bereich der Sensorelemente dargestellt, welcher in einem Abschnitt des Messkanals 7 zwei hintereinander angeordnete Sensorelemente 10 aufweist. Die Sensorelemente 10 bzw. deren Leiterbahnen 12 (welche im hier dargestellten Fall senkrecht zur Schnittebene weiterführen) sind durch die Zwischenschicht 13, welche eine elektrische Isolierung bewirkt, von der Messzellenwand 2 aus Metall oder einer Metalllegierung elektrisch isoliert.

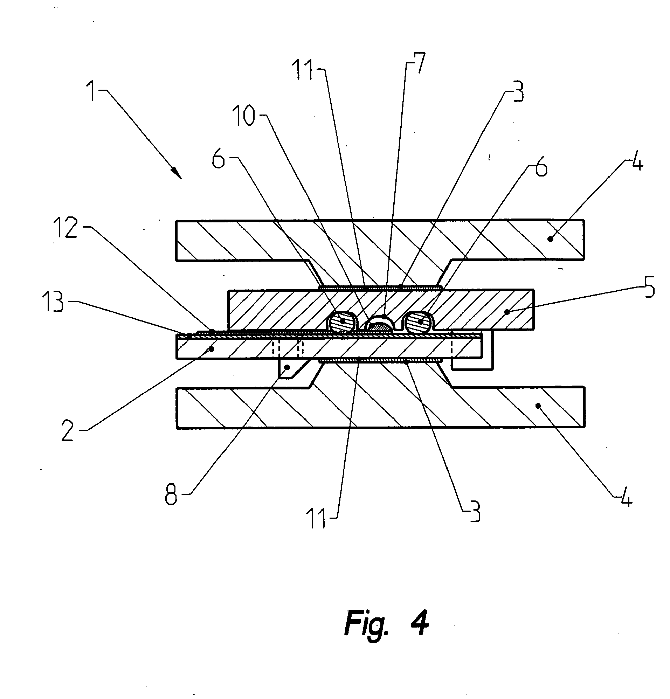

Weiters kann auch das Gehäuseoberteil als planare, gut wärmeleitende Messzellenwand 5 ausgeführt sein und unter Zwischenlage einer wärmeleitfähigen elastischen oder plastischen Schicht 11 mit einer thermostatisierten Auflagefläche 3 des Analysators in Kontakt stehen, sodass die Thermostatisierung der Messzelle 1 entweder nur über die Messzellenwand 5 des Gehäuseoberteils erfolgt oder über beide Messzellenwände 2 und 5, die - wie in Fig. 4 dargestellt - jeweils unter Zwischenlage einer wärmeleitfähigen, elastischen oder plastischen Schicht 11 an einer thermostatisierten Auflagefläche 3 des Analysators anliegen

Bei den Ausführungsvarianten gemäß Fig. 1, 3 und 4 besteht die der thermostatisierten Auflage des Analysators zugewandte planare Messzellenwand 2 bzw. das Gehäuseunterteil aus Metall oder einer Metalllegierung, sodass eine dünne Zwischenschicht 13, welche eine elektrische Isolierung bewirkt, zum Sensorelement 10 und dessen Signalableitung 12 nötig ist. Hierdurch ist eine direkte und schnelle Temperaturübertragung auf jene Bereiche möglich, die für die Sensorreaktionen wesentlich sind.

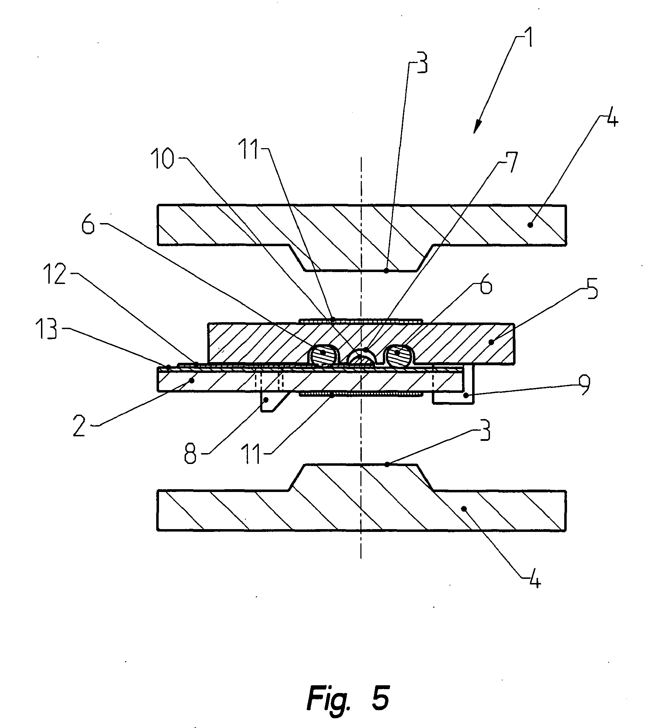

Fig. 5 zeigt die Ausführungsvariante gemäß Fig. 4 in einer Explosionsdarstellung mit den bevorzugt auf den Messzellenwänden 2 und 5 haftenden wärmeleitfähigen, elastischen oder plastischen Schichten 11, die rückstandsfrei von den Auflageflächen 3 des Analysators entfernbar sind.

Das Gehäuseunterteil bzw. die Messzellenwand 2 wird durch Laminieren oder Beschichtung, bevorzugt durch Siebdruck oder Schablonendruck, auf der einen Seite mit einem wärmeleitfähigen Silikon (z.B. Thermally Conductive RTV Silicone R-2930 von NuSil Technology CA 93013 USA oder ELASTOSIL® RT 675 von Wacker Silicones Deutschland) mit einer geeigneten Geometrie versehen und auf der anderen Seite ggf. elektrisch isoliert.

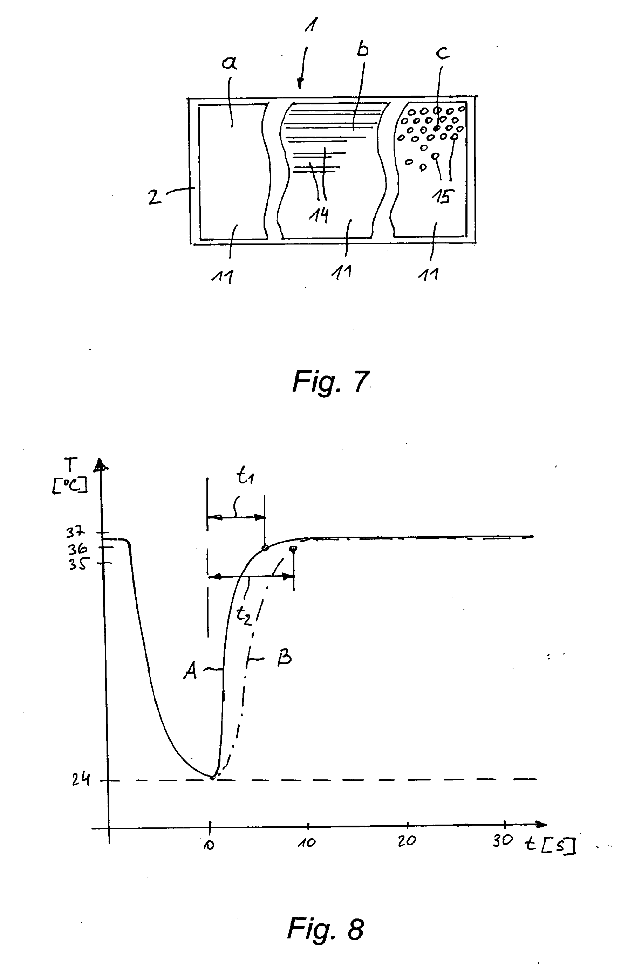

Wie in Fig. 7, einer Draufsicht auf das Gehäuseunterteil 2, in drei Ausführungsvarianten dargestellt, kann die wärmeleitfähige, elastische oder plastische Schicht 11 im Wesentlichen flächendeckend (Bereich a) aufgetragen sein oder zumindest an deren freien Oberfläche mit einer geeigneten Geometrie versehen sein, insbesondere eine Struktur, beispielsweise in Form von Streifen 14 (Bereich b) oder Noppen 15 (Bereich c), aufweisen.

Im Messdiagramm gemäß Fig. 8 wird die Einstellzeit t (in s) einer Messzelle dargestellt, die auf eine vorbestimmbare Temperatur T (in °C) thermostatisiert werden soll. Um eine Unebenheit zwischen der thermostatisierten Auflagefläche des Analysators und der Messzelle zu simulieren, wird zum Zeitpunkt t=0 ein kleiner Fremdkörper, im speziellen Fall ein Haar, eingebracht, wodurch sich bei der Messkurve B eine Einstellzeit t₂ ergibt, bis zu welcher 95% der Solltemperatur erreicht werden. Bei Verwendung der erfindungsgemäß wärmeleitfähigen, elastischen oder plastischen Schicht (Messkurve A) verringert sich die Einstellzeit wesentlich auf den Wert t₁.

## Patentansprüche

1. Vorrichtung zur Thermostatisierung einer Messzelle in einem Analysator, zumindest bestehend aus
der Messzelle (1) mit einem Messkanal (7), wobei im Messkanal (7) zumindest ein Sensorelement (10) angeordnet ist, und
dem Analysator, welcher eine thermostatisierbare Auflagefläche (3) aufweist,
wobei die Messzelle (1) in den Analysator austauschbar einsetzbar ist und zumindest in einem Kontaktbereich mit der thermostatisierbaren Auflagefläche (3) in Kontakt bringbar ist und wobei die Messzelle (1) eine zumindest in diesem Kontaktbereich im Wesentlichen planare Messzellenwand (2, 5) aufweist,
**dadurch gekennzeichnet, dass**
zur Verbesserung des Wärmeüberganges auf die Messzelle (1) eine wärmeleitfähige, elastische oder plastische Schicht (11) vorgesehen ist, welche zumindest im Kontaktbereich an zumindest einer Messzellenwand (2, 5) oder der thermostatisierbaren Auflagefläche (3) des Analysators haftet und beim Austausch der Messzelle (1) von der gegenüberliegenden thermostatisierbaren Auflagefläche (3) oder Messzellenwand (2, 5) weitgehend rückstandsfrei entfernbar ist
und/oder
dass die Messzellenwand (2), an deren dem Messkanal (7) zugewandten Innenseite das zumindest eine Sensorelement (10) angeordnet ist, zumindest im Kontaktbereich mit der thermostatisierbaren Auflagefläche (3) des Analysators aus einem wärmeleitfähigen Metall oder einer Metalllegierung besteht.

2. Vorrichtung nach Anspruch 1, **dadurch gekennzeichnet, dass** die wärmeleitfähige, elastische oder plastische Schicht (11) zumindest an deren freien Oberfläche eine Struktur, beispielsweise in Form von Streifen (14), Noppen (15) oder dgl., aufweist.

3. Vorrichtung nach einem der Ansprüche 1 bis 2, **dadurch gekennzeichnet, dass** die wärmeleitfähige, elastische oder plastische Schicht (11) aus einem wärmeleitfähigen Silikonmaterial besteht.

4. Vorrichtung nach einem der Ansprüche 1 bis 3, **dadurch gekennzeichnet, dass** die Messzelle (1) zwei oder mehrere planare Messzellenwände (2, 5) aufweist, die jeweils unter Zwischenlage einer wärmeleitfähigen, elastischen oder plastischen Schicht (11), welche zumindest im Kontaktbereich an der jeweiligen Messzellenwand (2, 5) oder der thermostatisierbaren Auflagefläche (3) des Analysators haftet und beim Austausch der Messzelle (1) von der jeweiligen gegenüberliegenden thermostatisierbaren Auflagefläche (3) oder Messzellenwand (2, 5) weitgehend rückstandsfrei entfernbar ist, an einer thermostatisierbaren Auflagefläche (3) des Analysators anliegen.

5. Vorrichtung nach einem der Ansprüche 1 bis 4, **dadurch gekennzeichnet, dass** das zumindest eine Sensorelement (10) als elektrochemisches Sensorelement ausgebildet ist und unter Zwischenlage einer Zwischenschicht (13), welche eine elektrische Isolierung bewirkt, an der Messzellenwand (2), welche aus einem Metall oder einer Metalllegierung besteht, angeordnet ist.

6. Vorrichtung nach einem der Ansprüche 1 bis 4, **dadurch gekennzeichnet, dass** das zumindest eine Sensorelement (10) als optisches Sensorelement ausgebildet ist und unter Zwischenlage einer Zwischenschicht (13), welche optisch transparent ist, an der Messzellenwand (2) angeordnet ist.

7. Messzelle, welche in einen Analysator austauschbar einsetzbar ist, mit einem Messkanal (7), wobei im Messkanal (7) zumindest ein Sensorelement (10) angeordnet ist und mit einem Kontaktbereich zu einer thermostatisierbaren Auflagefläche (3) des Analysators, wobei die Messzelle (1) eine zumindest in diesem Kontaktbereich im Wesentlichen planare Messzellenwand (2, 5) aufweist, **dadurch gekennzeichnet, dass** zur Verbesserung des Wärmeüberganges auf die Messzelle (1) eine wärmeleitfähige, elastische oder plastische Schicht (11) vorgesehen ist, welche zumindest im Kontaktbereich an zumindest einer Messzellenwand (2, 5) haftet und beim Austausch der Messzelle (1) von der thermostatisierbaren Auflagefläche (3) des Analysators weitgehend rückstandsfrei entfernbar ist.

8. Messzelle, welche in einen Analysator austauschbar einsetzbar ist, mit einem Messkanal (7), wobei im Messkanal (7) zumindest ein Sensorelement (10) angeordnet ist und mit einem Kontaktbereich zu einer thermostatisierbaren Auflagefläche (3) des Analysators, wobei die Messzelle (1) eine zumindest in diesem Kontaktbereich im Wesentlichen planare Messzellenwand (2, 5) aufweist, **dadurch gekennzeichnet, dass** zur Verbesserung des Wärmeüberganges auf die Messzelle (1) jene Messzellenwand (2), an deren dem Messkanal (7) zugewandten Innenseite das zumindest eine Sensorelement (10) angeordnet ist, zumindest im Kontaktbereich mit der thermostatisierbaren Auflagefläche (3) des Analysators aus einem wärmeleitfähigen Metall oder einer Metalllegierung besteht.

9. Messzelle nach Anspruch 8, **dadurch gekennzeichnet, dass** das zumindest eine Sensorelement (10) als elektrochemisches Sensorelement ausgebildet ist und unter Zwischenlage einer Zwischenschicht (13), welche eine elektrische Isolierung bewirkt an der Messzellenwand (2) angeordnet ist.

10. Messzelle nach Anspruch 8, **dadurch gekennzeichnet, dass** das zumindest eine Sensorelement (10) als optisches Sensorelement ausgebildet ist und unter Zwischenlage einer Zwischenschicht (13), welche optisch transparent ist, an der Messzellenwand (2) angeordnet ist.
